# EUROPEAN PATENT APPLICATION

(11) **EP 2 995 205 A1**
(43) Date of publication of application: **16.03.2016**
(21) Application number: 14184848.1
(22) Date of filing: 15.09.2014
(51) Int. Cl.: A23L 33/16, A23L 33/17, A23L 5/00, A23L 27/00, A61K 9/16, B01J 13/02

(54) **Compositions and methods for the maintenance of adequate iron intake in a mammal**

(71) Applicant: Anabio Technologies Limited, D2 Dublin (IE); Solvotrin Therapeutics Ltd., Cork (IE)
(72) Inventor: Doherty, Sinead, Dublin, D2 (IE); Ledwidge, Mark, Cork, Co. Cork (IE)
(74) Representative: Purdy, Hugh Barry

(57) **Abstract**

A preparation of microspheres comprises a multiplicity of discrete microspheres, in which the microspheres comprise iron entrapped within a protein matrix, and in which the protein in the protein matrix is at least partially denatured. The protein of the protein matrix comprises denatured, de-calcified, whey protein. Methods for making the preparation of microspheres are also described.

## Description

### Technical Field

The invention relates to compositions suitable for delivering iron to a mammal in a stable, bioavailable, well tolerated form. The invention also relates to methods for the avoidance and prevention of inadequate iron intake in a mammal. -

### Background to the Invention.

Iron is a vital nutrient essential for every human cell and is required for a number of highly complex mechanisms that are constantly occurring at a molecular level and are crucial to human life such as the transportation and storage of oxygen, in oxidative metabolism and in cellular growth and proliferation. [1] Iron is required for the production of red blood cells (haematopoiesis), plays a central role in brain development and function, is required for making genetic material (DNA) and it is necessary for healthy mineralisation and efficient repair of bones. [1-2] Iron is also essential for the immune function, as it stimulates the actions of inflammatory cytokines and for the manufacture of enzymes, with important roles in mitochondrial function. Other iron dependent enzymes act as antioxidants, for example, catalase and peroxidase, and protect tissues from free radicals, which can cause tissue and cell damage and impair health. [3]

Dietary iron exists in inorganic (non-haem) and organic (haem) forms. Approximately 3-4g of body content is iron, which correlates with a concentration of 40-50mg of iron per kilogram of body weight. Around 60% is present in the form of haemoglobin in circulating red blood cells; however, iron is incorporated into hundreds of different enzymatic and non-enzymatic proteins that are vital to a large range of physiological functions. [3-4] Iron is lost by the body through a number of different ways including urination, defecation, sweating and loss of old skin cells. Menstrual bleeding contributes to further loss of iron in women, which is why they have a higher need for iron than men. [3,5]

Iron metabolism is tightly regulated [6,7.] and absorption occurs mainly in the duodenum and upper jejunum, principally at the villus enterocytes in the intestine. Iron concentration is tightly regulated at several complex levels in the body and the typical pool in an adult of 3-4 g is augmented by absorption from the diet in the form of ferrous (Fe²⁺) or Heme. It is an active process, well characterized in the Fe²⁺ case and consists of specific transmembrane transport systems with three elements: an enzyme regulating the oxidative iron state, a specific transport protein complex, and regulatory proteins or co-factors. [8,9.]

Inorganic dietary iron is absorbed via the divalent metal transporter 1 (DMT1) on the apical surface of duodenal enterocytes. The accompanying ferrireductases reduce ferric to ferrous forms for absorption.[10] The DMT1 requires an enzymatic transmembrane co-factor which is stimulated by protons, possibly explaining the activity of DMT1 in the early part of the small intestine. On the other hand, haem iron is absorbed through a carrier protein, which is less well understood. However, an inducible haemoxigenase reduces haem iron before its release into the enterocyte cytosol. Accordingly, iron is found in the enterocyte cytosol in the ferrous form before being transported from the cytosol to the basolateral surface of enterocytes via ferroportin. At this surface, the accompanying membrane proteins hephaestin and/or cerulomodulin oxidize ferrous into ferric iron and are critical for the effective release of iron into the circulation bound to transferrin. [11]

Circulating ferric iron bound to transferrin provides a reservoir of iron deliverable to target cells and when bound to transferrin, ferric iron does not generate free radicals. Transferrin bound iron can then enter target cells using transferrin receptor type 1(TfR1)-mediated endocytosis, which is the major pathway of iron entry into these cells. As described above, the vast majority of intracellular iron is in erythrocyte haemoglobin and circulating red blood cell precursors, reticulocytes. Other target cells include: macrophages, which remove aging erythrocytes and support recycling of iron into the bone marrow; Browicz-Kupffer cells in the liver and hepatocytes, which release proteins regulating iron metabolism, including the main regulatory protein, hepcidin. Iron is usually stored intracellularly, predominantly in the liver, bone marrow and spleen cells, usually incorporated into ferritin where the bound iron is non-toxic. Ferritin can be exported extracellularly and the tissue expression of ferritin increases when there is too much iron. [6,7,10,11] Ferritin is also an acute phase protein, which can limit its usefulness as a single measure of iron storage.

The main iron transport pathways, both of which interact, are related to [1] iron absorption and transport between tissues and [2] iron transport within the cell between organelles and have been extensively reviewed. [6,11,12] The first, is largely regulated by hepcidin modulating ferroportin function. Hepcidin, is synthesized by hepatocytes, is elevated during the innate immune response and is highly conserved across the mammalian world. Hepcidin expression in hepatocytes is reduced by low iron stores, hypoxia and dysregulation of erythropoiesis. By interacting with ferroportin, hepcidin interacts with ferroportin, causing internalization of ferroportin, which disables its iron export function on the basolateral surface. This also can reduce the expression of DMT-1 and haem transporters,. [6,11,13-17]. In the gut, this results in internalization of iron in the enterocyte, which is then removed into the lumen of the gut when the enterocyte dies (1-2 days). In the body, this results in accumulation of iron from the circulation into the reticuloendothelial system and reduced iron availability to target tissues.[6,16]

Maintaining sufficient iron absorption is a major public health goal. According to the World Health Organisation, iron deficiency is the most common and widespread nutritional disorder in the world, affecting approximately 2 billion people. Furthermore, iron supplementation is associated with improved health and outcomes and under EU regulation 1924/2006 on Nutrition and Health Claims Made on Foods iron is recognized as helping to maintain normal cognitive function, energy-yielding metabolism, oxygen transport and immune function as well as reducing tiredness and fatigue. [18] Inadequate iron intake affects a large number of women and children in particular and is remarkable in being the only nutrient deficiency that is prevalent in industrialised countries. Iron status is associated with maternal and neonatal wellbeing and a recent large meta-analysis of 48 randomised trials and 44 cohort studies found a significant impact of daily preterm iron supplementation on birth weight. [19] Inadequate iron intake in older age groups generally occurs in tandem with chronic disease. It is the major cause and pre-cursor to anaemia and is linked with renal, gastrointestinal, cardiovascular disease and diabetes.

Inadequate iron intake is also highly prevalent in developing economies and is associated with poor maternal and child health. Recently, Persson and colleagues reported a prospective, randomized, double-masked assessment of early invitation (9 weeks' gestation) versus usual invitation (20 weeks' gestation) to partake in a programme with various supplement formulations including iron. [20] There were no differences in maternal haemoglobin or birthweights for the 60 vs 30 mg iron formulations. Interestingly, mean maternal hemoglobin level was slightly lower in the early vs. usual invitation groups (114.5 vs 115.4 g/L). Previously, a dose response association between iron ingested and haemoglobin was shown to plateau at approximately 1800 mg ingested iron - and 30mg daily iron the micronutrient supplement group in the study of Persson et al. resulted in average iron intake of 2190 mg. [21] Inadequate iron intake is also a concern in areas where infectious diseases such as malaria, HIV, tuberculosis and schistosomiasis are endemic [22] because of the altered metabolism of iron by the monocyte/macrophage system during chronic infection.

Iron bioavailability can vary from 1% to more than 15 [23,24] The main factor influencing iron absorption is the amount of iron stored in the body. Iron absorption in the gastrointestinal (GI) tract increases when the body's iron stores are low and decreases when there's a sufficient amount of iron. An increased rate of red blood cell production can also stimulate iron uptake several-fold [4,25].

In adults, absorption of dietary iron is approximately 6% for men and 13% for non-pregnant females in their childbearing years [26]. The higher absorption efficiency in women is due to their lower iron stores as a result of menstruation and pregnancy.

The bioavailability of iron depends on dietary composition. Heme iron is two to three times more absorbable than non-heme iron. Red blood cell formation and destruction is responsible for most of the iron turnover in the body. In men, around 95% of iron is needed for the production of red blood cells. Most of this iron comes from the breakdown of red blood cells while 5% comes from dietary sources [25].

There are important limitations with medicinal approaches to managing inadequate iron intake. While the world health organization advocates use of ferrous sulfate, [27] the absorption of iron from all oral iron salts (sulfate, gluconate, fumarate) is poor resulting in lack of efficacy and slow restoration of iron stores. Since availability of iron from oral formulations is poor, where only 6-15% of the total oral iron dose is absorbed,[24] the balance is excreted in the faeces and causes stool discolouration and gastrointestinal disturbances. A possible explanation for this high incidence of gastrointestinal side effects is the resulting local oxidative stress in the gut mucosa, causing abdominal pain as well as other gastrointestinal side effects. [28] Constipation (35%), nausea (13%) and vomiting (8%) are common in treatment studies with high doses of oral iron. [29] Taking oral iron with food can reduce stomach upset, but this also reduces further the iron absorption. In pregnancy, women frequently attribute these side effects to ingestion of their oral iron and adherence to the regimens is often poor resulting in a suggestion that intermittent oral iron supplementation be used (dosing 3 times per week) to limit these side effects and improve adherence. [30] Protecting the iron release using enteric coating and sustained release formulations can improve the tolerability, but the efficiency of iron absorption is reduced. The use of intravenous formulations for severe iron deficiency states is expensive, unpleasant, inconvenient and carries significant risk of adverse reaction, frequently requiring post-administration observation of the patient.

Therefore, medicinal approaches to maintenance of adequate iron intake carry an unacceptable risk of adverse effect for many consumers. Accordingly, supplement formulations with low doses of iron have been developed to avoid these adverse effects. However, the supplement formulations suffer from the limitation of having inadequate iron absorption. For example, a widely used solution of ferrous sulfate has reported bioavailability of iron in non-pregnant and pregnant women of 14% to 28% using a daily dose of 25mg. [31] Given the daily requirement for iron exceeds this several fold, especially in pregnant women, these formulations do not address the consumer need. A further limiting factor associated with oral liquid formulations of iron sulfate is the poor taste, again limiting adherence to its use.

In summary, the accepted health benefits of iron-fortified foods include improvement in normal cognitive function, energy-yielding metabolism, oxygen transport and immune function as well as reducing tiredness and fatigue. For the vast majority of consumers, medicinal products are inappropriate and are associated with high risk of adverse effects. For currently available supplement products that avoid these adverse effects, the efficacy is often poor and/or unproven and there may be other adverse effects such as poor palatability. The unmet consumer need for new supplement products to effectively provide adequate iron intake in a well-tolerated manner is high.

Borch-Iohnsen et al. [32] in studies in humans observed a difference in iron absorption rates between two groups of subjects, with normal iron stores and low or empty iron stores. Greater iron absorption rates were observed for the latter group. Zhang and Mahoney [33] found that differences in maximal iron bioavailabilities among the different iron sources used (ferric chloride, iron-casein, ferripolyphosphate-whey protein, and iron-whey protein complexes) and between fortified cheeses and fortification iron sources were not significant. Studies of Hurrell et al. [34] on effects of casein and whey protein on Fe absorption using standardised liquid formula meal in humans showed that absorption of this element was decreased compared with the protein free formula. A 1.7-fold improvement in Fe absorption was seen when a 36% enzyme-hydrolysed whey protein was used compared with the non-hydrolysed protein, however this value was not better than the control and the authors concluded that whey proteins have inhibitory effects on Fe absorption, Remondetto et al. [35] used practically pure beta-lactoglobulin to form "particulate" and "filamentous" gel structures by modulating the amount of iron sulfate (10 mM versus 30 mM Fe²⁺ to form filamentous and particulate structures, respectively) added to the beta-lactoglobulin dispersions. This work concentrated on studying the structural differences between the gels produced in terms of Fe release at different pH values and in the presence of enzymes as well as in vitro estimation of Fe absorption of Fe in a CACO-2 cell line. This work presents that the filamentous structures were better in vitro dissolution studies than the particulate systems due to Fe being bound to the exterior of the linear aggregates of filamentous gels, thus facilitating its release. It was suggested, but not demonstrated in vivo, that greater bioavailability of Fe released from filamentous gels may be obtained compared to particulate gels. It needs to be highlighted that no in vivo studies were performed by Remondetto et al.

Nakano et al. [36] performed in vivo study on Fe absorption using Fe-deficient rats. An iron-fortified whey protein (Fe-FWP) versus heme iron was investigated however no statistically significant apparent iron absorption rates from these two sources of Fe were measured. The authors state that higher solubility of Fe from the Fe-FWP in comparison to hemin was measured in the intestines.

Ou et al. [37] investigated the effects of neutrase, alcalase and papain hydrolysis on whey protein concentrates on in vitro iron uptake by Caco-2 cells. It was concluded that neutrase and papain treated whey protein concentrates are able to improve the amount of iron transported and retained by the cells and the former system appeared to be a promising facilitator of iron absorption. The authors hypothesise that relatively low molecular weight peptides formed upon hydrolysis of whey protein concentrates could be mediators of iron bioavailability. Again, no in vivo data are present to endorse iron bioavailability in mammals.

EP2292102 A1 describes a stable microcapsule comprising a protein-polysaccharide shell matrix and a core, said core comprising an emulsion of an aqueous dispersion or solution of at least one mineral salt in an edible oil, said mineral salt being entrapped in the core of the microcapsule and said mineral salt being insulated from the protein-polysaccharide matrix.

EP 0504387 B1 describes a process of producing microcapsules by dispersing the core material in a protein slurry, heating the slurry to create a protein melt, denaturing the protein melt, thereby causing protein particles to encapsulate the core material dispersed in the melt and comminuting the encapsulated core material to provide microcapsules.

US6139882 describes bicarbonate and/or carbonate iron-whey-proteolysate complex for taste masking.

It is an object of the invention to overcome at least one of the above-referenced problems.

### Statements of Invention

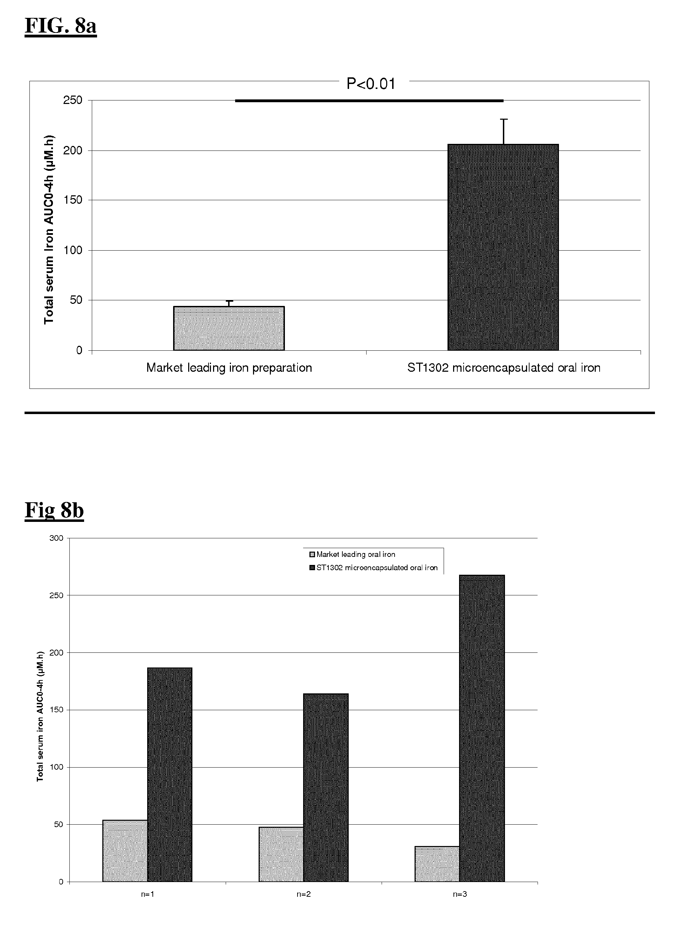

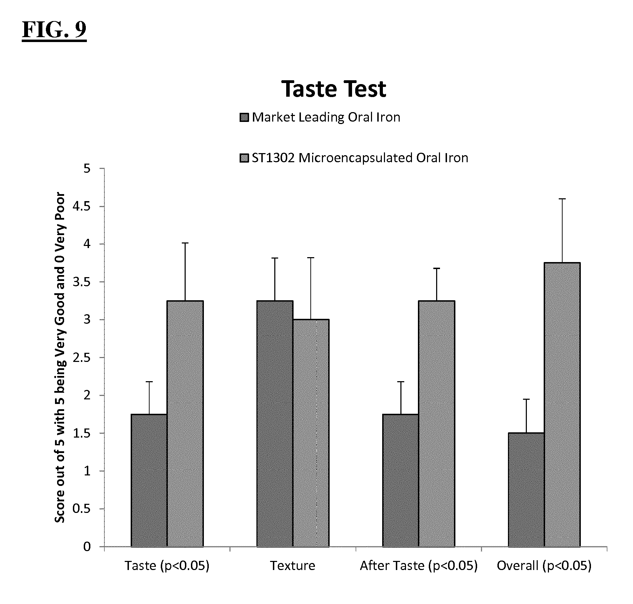

The Applicants have surprisingly discovered that many of the problems associated with delivery of iron to humans in a stable and bioavailable form can be overcome by employing microspheres formed from an at least partially denatured protein matrix having iron entrapped within the matrix. The Applicant has discovered that such iron containing microspheres, in which the matrix of the microsphere is at least partially formed from denatured whey protein, and when delivered to a mammal via the oral route, results in improved stability of the active agent during storage and oral delivery, and greater bioavailability and absorption of the iron *in-vivo* (Fig.8). Additionally the entrapment reduces the adverse effects and improves palatability (Fig. 9). For the vast majority of consumers, this provides a way of maintaining adequate iron intake without the adverse effects of medicinal products. For those taking available supplement products, it provides a way of more effectively maintaining iron intake without other adverse effects such as poor palatability associated with iron intake.

Thus, in a first aspect, the invention provides a preparation of microspheres comprising a multiplicity of discrete microspheres, in which the microspheres comprise iron entrapped within a protein matrix, and in which the protein in the protein matrix is at least partially denatured.

Typically, the protein in the protein matrix has been subjected to divalent metal ion removal process. Preferably, the protein has been de-calcified.

Suitably, the protein in the protein matrix comprises whey protein, another milk protein, or pea protein. Preferably the protein is whey protein, more preferably denatured whey protein, and ideally de-calcified, denatured whey protein.

Suitably, the microspheres comprise 0.1% to 5% iron (dry weight %) Preferably, the microspheres comprise 0.1% to 2.0%, 0.1% to 1.5%, 0.1% to 1.0%, 0.25% to 0.75% iron. The term "dry weight %" as used herein means the % by weight of iron in the microspheres when in a dry form (i.e. the % by weight of iron in spray-dried microspheres).

Typically, the iron is ferrous (II) iron, ideally selected from iron sulfate, iron chloride, iron ascorbate, and an iron carbohydrate complex.

In another embodiment, the iron is a ferric (III) iron, optionally selected from iron (III) maltose or iron (III) maltol.

Preferably, the microspheres comprise a coating of a citrate, phosphate, or ascorbate salt.

Ideally, the protein of the protein matrix comprises denatured, de-calcified, whey protein.

The invention also provides a method of treating or preventing iron deficiency in a mammal, comprising the steps of administering a preparation of microspheres according to the invention to the mammal. Typically, the method is non-therapeutic, and relates to treatment of non-clinical iron deficiency by means of iron supplementation.

The invention also relates to a method of maintaining adequate iron intake in a mammal comprising the step of administering a preparation of microspheres according to the invention to the mammal.

The invention also relates to a preparation of microspheres according to the invention, for use as a dietary supplement in preventing the development of iron deficiency or an iron deficit in a healthy mammal.

In the methods of the invention, the preparation of microspheres are administered at a dosage sufficient to deliver from 5-100mg, 5-50mg, 5-30mg, 5-20mg, 10-100mg, 10-50mg, 10-40mg, or 10-30mg iron to the mammal daily. Thus, if the target iron dosage is 30mg per day, and the microcapsules comprise 0.75% iron (dry wt %), this would imply administering 40g of microcapsules per day. The invention also relates to a preparation of microspheres of the invention provided in a unit dose form and comprising 10-40mg, 10-30mg, 20-30mg of iron per unit dose. The unit dose may be a tablet, capsule, sachet, beverage or the like.

The invention also relates to a composition comprising a preparation of microspheres in accordance with the invention, in which the composition is selected from a food or beverage product, and animal feed, or a health supplement for humans or animals.

The invention also relates to a method for making a preparation of microspheres according the invention, comprising the steps of:
a. preparing an iron-containing solution;
b. separately preparing a suspension of at least partially denatured protein;
c. delivering the solution and suspension to a nozzle;
d. simultaneously extruding the solution and suspension through the nozzle in a laminar jet in a manner that produces microdroplets; and
e. curing the microdroplets in an acidification bath such as an acetate bath to generate the microspheres comprising the iron entrapped within a crosslinked protein matrix.

The invention also relates to a method for making a preparation of microspheres according to the invention, comprising the steps of:
a. extruding a suspension of at least partially denatured protein through a vibrating nozzle to form a laminar jet in which break-up of the extruded laminar jet into microdroplets is induced by applying a sinusoidal frequency with defined amplitude to the nozzle; and
b. curing the microdroplets in an iron containing acidification bath to generate the microspheres comprising the iron entrapped within a crosslinked protein matrix.

Preferably, the acidification bath comprises an acetate buffer having a pH of 3.5 to 4.5, a concentration of 0.15M to 0.25M, and optionally a temperature of 40-50°C.

Typically, the microspheres are immersed in a buffer comprising a citrate, ascorbate or phosphate salt for a period of time sufficient to allow coating of the microspheres with a citrate, ascorbate or phosphate salt.

Typically, the microspheres are dried. Various methods for drying microsphere preparations will be known to the person skilled in the art, including spray drying. Suitably, the microsphere preparation will be milled after drying.

Preferably, the protein suspension or solution comprises 5-15 % de-calcified denatured whey protein (w/v).

In another aspect, the invention relates to a microsphere, or a preparation of microspheres, in which the microspheres comprise a crosslinked and polymerized matrix formed from denatured de-calcified protein having iron entrapped within the matrix (w/w).

Suitably, the microspheres comprise 0.1% to 5% iron (dry weight %) Preferably, the microspheres comprise 0.1% to 2.0%, 0.1% to 1.5%, 0.1% to 1.0%, 0.25% to 0.75% iron.

Typically, the microspheres have a spherical shape, suitably a mean diameter of less than 250 microns, and suitably a homogenous size distribution.

Preferably, the microsphere of the invention is coated with a citrate salt, preferably tri-sodium citrate, or a phosphate salt, preferably di-sodium phosphate, or an ascorbate salt. This has been found to improve bio-absorption compared with equivalent non-coated microspheres.

In another aspect, the invention relates to a microsphere of the invention, or a preparation of such microspheres, for use in a method of treating or preventing inadequate iron intake in a mammal, typically a human.

In another aspect, the invention relate to a composition comprising microspheres of the invention.

One of the challenges with delivering iron in whey protein microspheres is maintaining the iron in a stable form. The Applicant has discovered that ferrous iron is stabilized at a low pH, typically less than 4.5, while protein solutions are required to have a neutral pH to maintain a low viscosity and avoid flocculation. This necessitates keeping the ferrous iron and protein separate until just prior to formation of protein ferrous iron microdroplets. As the microdroplets are immediately cured in an acidification bath, the ferrous iron is thus instantaneously entrapped in a protein matrix in a stable form suitable for long-term storage.

Thus, in another aspect, the invention provides a method for making iron containing microspheres, comprising the steps of: preparing a ferrous iron containing solution; separately preparing a de-calcified denatured whey protein suspension; delivering the solution and suspension to a vibrating nozzle, in which the solution and suspension are admixed at or just prior to the nozzle; simultaneously extruding the admixed solution and suspension through the vibrating nozzle to form a laminar jet in which break-up of the extruded laminar jet into microdroplets is induced by applying a sinusoidal frequency with defined amplitude to the nozzle; and curing the microdroplets in an acidification bath to generate the microspheres comprising the ferrous iron entrapped within a crosslinked de-calcified denatured whey protein matrix.

Another way of making the microspheres of the invention comprises extruding the de-calcified denatured whey protein suspension into an acidification bath that includes ferrous iron. Thus, the invention also provides a method for making ferrous iron containing microspheres, comprising the steps of: extruding a de-calcified denatured whey protein solution/suspension through a vibrating nozzle to form a laminar jet in which break-up of the extruded laminar jet into microdroplets is induced by applying a sinusoidal frequency with defined amplitude to the nozzle; and curing the microdroplets in a ferrous iron containing acidification bath to generate the microspheres comprising the ferrous iron entrapped within a crosslinked de-calcified denatured whey protein matrix.

Preferably, the method includes an additional step of immersing the microcapsules in a buffer comprising a citrate or phosphate salt such that the resultant microspheres are coated with a citrate or phosphate salt.

### Brief Description of the Figures

**Fig. 1** illustrates a system for preparing ferrous iron containing de-calcfied denatured whey protein microspheres of the invention; and
**Fig. 2** illustrating Beta-Lactoglobulin protein stands in the matrix of ferrous sulfates micro-capsules. The bar represents 200 nm length.
**Fig. 3****:** Pore size analysis and frequency in ferrous microspheres. It is clear that the pore size and width increases as a result of the citrate treatment, which is advantageous for mucoadhesion to the duodenum and upper jejunum wall.
**Fig. 4****:** Ferrous sulfate capsules generated for duodenal and upper jejunum digestion and ferrous release. Bar represents 50 microns (left image) and 100 microns (right image).
**Fig. 5****:** Atomic Force Microscopy calibration grid to estimate the pore distribution (grid) and distribution (red columns) as a function of microspheres surface area.
Fig. 6: Atomic Force Microscopy image of microsphere porous surface after acetate and citrate treatments
Fig. 7: Illustrate the presence of large, deep pore (green line) on the surface of a microsphere after acetate and citrate polymerization. It is evident that capsule treatment with acetate alone will not generate large pores that are necessary for capsule mucoadhesion near the duodenum and upper jejunum.
Fig. 8a/b: (a) Total serum iron AUC₀₋₄ₕ (µM.h) of soluble oral iron formulation and test formulation at equimolar elemental iron dose (25mg ± 0.334mg) observed in fasting state (n=3) in a randomised crossover study. Figure a shows aggregated data and figure b individual participant data.
Fig. 9: Clinical assessment of soluble oral iron formulation and test formulation at equimolar elemental iron dose (25mg, n=3 per treatment) in a randomised crossover study.

### Detailed Description of the Invention

In this specification, the term "microsphere" should be understood to mean a particle, typically a generally spherical particle, having a mean particle spherical diameter of 60-250µm, 60-150µm, 60-120µm, and preferably 60-100µm as determined using the laser diffraction technique and comprising a gelled filamentous network formulated from at least partially denatured protein material for the space-filling effect of iron encapsulation. The term "de-calcified" as applied to microspheres should be understood to mean that the microspheres comprise less than 1.0%, preferably less than 0.1%, and ideally only trace amounts of calcium (dry wt%) as determined by standard HPLC methodologies.

Particle size analysis was performed by dispersing the microencapsulated iron in Milli-Q water after which size distribution was measured at 25°C in triplicate for each manufactured batch by dynamic light scatter (Mastersizer 2000, Malvern, Worcestershire, UK). Microsphere diameter and size distribution were determined as a function of polymerization time, iron concentration and pH of whey protein suspension. In this way, the optimum conditions or reproducible microsphere size was determined. Encapsulated iron particles had a spherical shape with a specified size ranging between 0.2-2000 µm. For the purpose of this invention, the particle size distribution was measured by dispersity (D, formerly polydispersity index) which is a measure of the distribution of particle size in a fluid; the higher the value of dispersity the lower the uniformity in particle size. The range for different iron formulations within this invention was 0.25-0.5 (Mastersizer 2000).

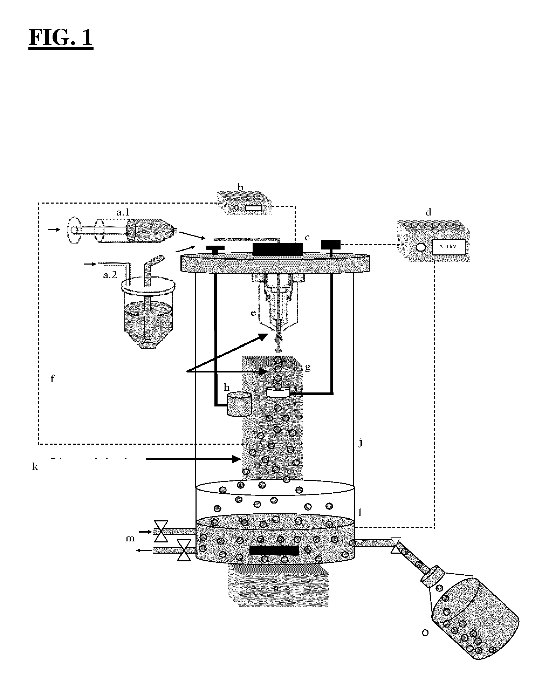

Production of mono-dispersed microspheres with a very narrow size distribution typically involves the generation of a steady flow of the whey protein polymer through the nozzle at controllable rates, which will provide optimum breakup of the extruded liquid jet into droplets optimally suitable for ferrous encapsulation (Fig. 1). In order to facilitate extrusion of a continuous stable laminar liquid jet through the nozzle, a system pre-requisite outlines the use of a high flow rate to surpass the viscosity and /or the surface tension of the denatured de-calcified whey protein solution/ suspension. Nonetheless, the flow rate must not be excessively high in order to avoid inefficient jet-breakup and enhanced impact forces on the whey protein droplets when entering the acetate gelling bath with predictable deformation [38] and increased probability of coalescence [39].

Microspheres size can be varied within a certain range by regulating the frequency of vibration and /or the flow rate of the protein with higher frequencies and lower jet velocities enabling the generation of smaller whey protein-iron droplets. Nonetheless, the main factor governing iron microsphere size using the present invention is the nozzle diameter, either single or concentric whereby the final microsphere diameter is approximately 1.25X the size of the chosen nozzle, using this specific technique.

Optimal breakup of the jet results in the formation of a single bead chain, which can be monitored using a stroboscopic lamp (Fig. 1g) placed directly behind the protein droplet chain providing visualization of individual protein-iron drops during breakup. Droplet coalescence in the laminar jet stream will result in loss of monodispersity of iron microspheres and an increase in the microsphere size deviation. [40]

Suitably, to avoid coalescence in droplets in the air, the protein suspension is adjusted to a pH above the isoelectric point (*pI*) most suitable to induce a strong negative charge on the denatured de-calcified whey protein droplets. This generates droplet repulsion and dispersal of the chain into a cone-like shape (Fig. 1k) due to Coulomb forces. [40] In this way, the use of an electrostatic system is not essential to enable droplet dispersion. One embodiment of the invention, outlines the need for strong negative whey protein charges to allow tangential deflection of the jet stream during breakup. A person skilled in the art would assume the requirement for electrostatic potential; however, the negative charge on the denatured de-calcified whey protein eliminates the need for electrostatic systems during the encapsulation process. Hence, this process avoids the formation of "doublet" microspheres and enlarges the drop-zone in the hardening acetate bath solution (Fig. 1l), resulting in mono-dispersed, homogenous and spherical iron microspheres.

Suitably, when the protein is whey protein, the whey protein suspension has a pH value above 4.8, typically above 5.2, and ideally above 5.4. Having a strong negative charge on the denatured de-calcified whey protein droplets has been shown to provide for improved microsphere formation. Thus, the pH of the whey protein "feed" solution/suspension to the nozzle is important to help generate optimum microsphere morphology at a reproducible size.

Suitably, the microspheres are capable of binding to the intestine, especially the lumen of the duodenum or upper jejunum, and releasing the iron at the lumen. In one embodiment, the microspheres have a porous surface, as determined using AFM techniques.

Atomic force microscopy (AFM) images were also obtained using Asylum Research MFP-3D-AFM (Asylum Research UK Ltd., Oxford, UK) to investigate the frequency of pores on the surface of the microspheres. The microcapsules are poured onto microscope glass slides, incubated for slide adsorption for approx. 20 minute, washed with ultraclean water and dried in a nitrogen flow leading to a linear surface coverage of microspheres on the slide. Suitable microsphere samples could then be chosen in transmitted light DIC contrast prior to AFM scanning. The AFM head is then placed onto a Zeiss Axiovert 200 inverted optical microscope. AFM analysis was relatively slow since microspheres were delicate and easily damaged mechanically if the force exerted by the AFM tip was too high. Microscope images were obtained in intermittent contact mode with standard silicon non-contact cantilevers (NCH) provided by Asylum Research, UK. Resonance frequency was approx. 300 kHz and spring constant of 40N/m. Digital resolution was 512 x 512 pixels. The tip radius of cantilever was less than 10 nm and the opening angle between 40° and 50°. The applied force between the cantilever tip and ferrous capsules was minimised in order to alleviate the influence of the AFM tip on the capsule surface topography during pore scanning. Measurements were started by scanning a random area of 1 m, with subsequent zoom to 20nm. These images were used to evaluate the morphology of microsphere pores. Scan size was decreased gradually until pores could be viewed clearly. Iron-whey protein microspheres were analysed and scanned using typical working distance of 2-8 nm for visualization of the pores.

Ideally, the microspheres have a coating of a promotor capable of opening tight junctions in epithelial cells. Preferably, the promotor is a citrate salt, ideally tri-sodium citrate, or a phosphate salt, ideally di-sodium phosphate, or another salt such as an ascorbate salt. This additional coating was required to enhance iron bioavailability Typically, the microsphere of the invention comprises (as a dry weight %):
- 75-95% denatured, optionally de-calcified, whey protein; and
- 0.1-5.0% iron.

Preferably, the microsphere of the invention comprises (as a dry weight %):
- 85-95% denatured, optionally de-calcified, whey protein; and
- 0.1-5.0% iron.

Suitably, the microsphere of the invention comprises (as a dry weight %):
- 75-95% denatured, optionally de-calcified, whey protein isolate; and
- 0.1-5.0% iron.

Ideally, the microcapsule of the invention comprises (as a dry weight %):
- 85-95% denatured, optionally de-calcified, whey protein isolate ; and
- 0.1-5.0% iron.

The term "as a dry weight %" means as a weight% of the microcapsule in a dry format, for example a microcapsule in a spray dried format.

In this specification, the term "at least partially denatured" should be understood to mean a protein preparation that comprises at least 10%, 20%, 30%, 40% or 50% denatured protein as determined by Ellman's technique (detailed below).

In this specification, the term "denatured whey protein" should be understood to mean a whey protein preparation that comprises greater than 96%, 97%, 98% or 99% denatured whey protein as determined by Ellman's technique (detailed below), and which is substantially free of non-denatured whey protein and ideally also substantially free of hydrolysed or partially hydrolysed whey protein. Hence, the use of fully denatured protein is the optimized encapsulation material to be used for the delivery of ferrous iron in microspheres. The denatured whey protein is generally a denatured whey protein concentrate or denatured whey protein isolate. Methods for denaturing whey protein will be known to those skilled in the art, and include heat denaturation and pressure-induced denaturation. In a preferred embodiment of the invention, the whey protein is heat denatured at a temperature of 70°C to 140°C, preferably 78°C to 121°C. Suitably, when the whey protein has a pH within the 5-9 range and a concentration of greater than 7%, it is heated at a temperature of greater than 70°C for more than 15 minutes. Typically, when the whey protein has a pH within the 5.5-7.5 range and a concentration of greater than 9%, it is heated at a temperature of greater than 70°C for more than 25 minutes is heat denatured for a time of between 30 and 50 minutes. Usually, the whey protein is agitated during denaturation.Normally, the β-lactoglobulin has a degree of denaturation of at least 60%, 70%, 80%, 90% or 95% , preferably greater than 80%, as determined using HPLC technique.

Ellman's reagent (2-nitro-5-mercaptobenzoic acid, DTNB) was utilised to determine the number of free thiol groups available after protein denaturation. The presented assay was performed in the absence of any reagents such as urea or SDS, because under these conditions, only the thiol groups of interest, those of the surface of the protein aggregate, were determined for encapsulation purpose. The number of thiol groups free for ferrous encapsulation was calculated using a molar extinction coefficient for DTNB of 13,600 M⁻¹ cm⁻¹.

Generally, denatured protein is first "activated" by heat to fully denature and unfold its globular structure. This creates a protein network, driven by physical interactions for iron encapsulation.. Hence, the polymerisation conditions and residence time are highly important for the rigidity and strength of denatured de protein microspheres for successful iron delivery to the duodenum and upper jejunum of mammals.

Typically, the protein employed in the process of the invention has at least 90%, 94% or 98% protein content (on a moisture and fat free basis).

Suitably, the protein is de-calcified. In this specification, the term "de-calcified" as applied to the protein should be understood to mean that the protein has most of its calcium replaced with monovalent cations, as determined using a technique described in http://en.wikipedia.org/wiki/Cation-exchange_capacity. Preferably, the protein, ideally denatured protein, comprises less than 1% calcium, more preferably less than 0.1 % calcium, and ideally only trace amounts of calcium (w/v). Many methods of de-calcification of protein will be apparent to those skilled in the art, including (a) cation exchange, (b) acidification to pH 4.6-7 with subsequent dialysis and/or ultrafiltration and/or diafiltration, and or (c) through use of a calcium chelating or sequestering agent.

Preferred denatured whey protein (i.e. WPI) for encapsulation purposes have calcium removed by a cation exchange method for efficient encapsulation of ferrous compounds for improved bioavailability in the blood serum. Preferably, the cation exchange has been performed on a resin bearing strongly acidic groups, preferably sulfonate groups. The functional groups are sulphonic acid groups that can be obtained in the Na⁺ form or alternatively converted to the K⁺ form. The use of the Na⁺ or K⁺ form is preferred.

Typically, the whey protein (i.e. WPI) applied to the cation exchanger preferably has the pH in the range of 5.0 - 8.0, more preferably 5.5-6.5. Once whey protein has passed through the column its pH increases. If it increases above 7.0, it will generally be adjusted to about 6.5-7.0 to make it more possible to mask the taste of ferrous compounds during proposed encapsulation procedures.

The treatment of whey protein (i.e. WPI) for use as an encapsulation agent for iron, typically ferrous iron, firstly requires the removal of calcium by acidification, followed by dialysis and ultrafiltration, the pH is adjusted to be in the range 4-8, preferably 4.4-7.0, more preferably 4.6-6.8. The preferred ultrafiltration membrane requires a nominal molecular weight cut off of 10,000 Daltons or less. For encapsulation purposes, it is preferred to neutralise (pH 5-7) the solution and filter the solution (through 0.45 micron) to remove any denatured material generated during the powder production. A chelating agent such as citric acid, citrate or typical food acidulants can also be added in order to remove the calcium. In general, chelating agents may be used before, during or following ultrafiltration or independently of an ultrafiltration or diafiltration. However, for this application, it is preferable to remove calcium using a chelating agent such as citrate before the ultrafiltration process.

Suitably, the concentration of the at least partially denatured protein solution/suspension is from 4 to 30%, preferably from 7 to 30%, and ideally from 9 to 16% (w/v). Typically, the protein is whey protein, ideally denatured de-calcified protein, optionally in the form of a dispersion, in which partial gelation of the β-lactoglobulin produces a protein agglomerates. Typically, the suspension is subject to a filtration process prior to being delivered to the nozzle. In one preferred embodiment, the suspension is passed through a plurality of filters having a gradually decreasing pore size. Ideally, the final filter has a sub-micron pore size, for example 0.1 to 0.9 microns. Ideally, the second liquid stream has a pH of 4.8- 6.8, the pH ideally suited for the generation of a negative charge on the denatured whey protein droplets during extrusion.

Ideally, the at least partially denatured (optionally de-calcified) whey protein employed in the process of the invention has a β-lactoglobulin content of at least 30%, 40%, 50%, 60%, 70% or 80% (w/w).

In this specification, the term "ferrous iron" should be understood to mean iron in a form having a +2 oxidation state, for example an iron II compound. Examples of ferrous iron include iron II salts such as ferrous sulfate, ferrous chloride, ferrous nitrate or iron oxides, or heme iron. Preferably, the ferrous iron is an iron II salt. For the purpose of this invention, the details for the spectrophotometric technique are provided for the determination ferrous iron concentration in microspheres (via calorimetric analysis). Microspheres are reacted with specialized reagent to form a coloured microsphere with complexed ions. The intensity of the coloured species is measured using a Cary spectrophotometer. A calibration curve (absorbance versus concentration) is constructed for iron +II and the concentration of the unknown iron in microspheres samples. This methodology is based on the change in the intensity of the colour of a solution with variations in concentration. Colorimetric methods represent the simplest form of ferrous iron analysis for this present invention.

It is possible to determine iron concentrations at the ppm level. 2, 2'-Bipyridyl (bipy), Mw = 156.20, forms an intensely red complex with iron (II) which may be exploited to determine iron concentrations in the ppm range. The reaction is:

3bipy + Fe²⁺ <===> Fe(bipy)3²⁺

The complex formed with encapsulated ferrous iron conforms to an octahedral geometry with coordinate covalent bonds being formed between the adjacent sp³d² hybrid orbital's of the Fe²⁺. The complex is chiral; there are left-handed and right-handed non-superimposable optically active forms. The molar absorptivity of the iron-bipyridyl complex is 8650 L/mol/cm at the wavelength of maximum absorbance. The complex forms rapidly, is stable over the pH range 3 to 9, efficiently measures iron(II) concentrations in the range of encapsulation utilized in the present invention, and also lowers levels such as 0.5 to 8 ppm.

Suitably, the ferrous iron solution has a pH of less than 5, and ideally of less than 4.5.

In this specification, the term "citrate, ascorbate or phosphate salt" should be understood to mean sodium citrate, ideally tri-sodium citrate or alternatively ascorbic acid, and phosphate salt should be understood to mean di-sodium phosphate. The citrate, ascorbate and phosphate salts have been found to significantly improve the absorption of ferrous iron in the mammalian gastrointestinal tract. Without being bound by theory, it is believed that this is due to the citrate, ascorbate or phosphate acting as an absorption promotor, opening tight junctions in epithelial cells lining the lumen of the GI tract, and thereby facilitating delivery of the ferrous iron compounds. Citrate also binds calcium, a non-competitive inhibitor of the divalent metal transporter DMT1. Suitably, the citrate or phosphate bath has a salt concentration of 0.2 to 0.4M, typically 0.25 to 0.35M, and ideally about 0.4M. Typically, the citrate or phosphate bath has a pH of 4-6, suitably about 4.5 to 5.5. Generally, the citrate or phosphate bath has a temperature of 15-25°C, typically 18-22°C.

In this specification, the term "acidification bath" refers to a bath composed of an organic acid, for example acetic acid, that has a pH sufficiently low to cure the microdroplets to form solid microspheres. The bath is typically free of calcium ions. Suitably, the bath has an organic acid concentration of 0.1 to 0.3M, typically 0.15 to 0.25M, and ideally about 0.2M. Typically, the bath has a pH of 3 to 4.5, suitably about 4. Generally, the bath has a temperature of 40-50°C, typically about 45°C. Typically, the acidic curing solution comprises a surfactant to prevent or inhibit agglomeration of the formed microspheres. Suitably, the surfactant is a polysorbate surfactant, ideally Tween 20.

Suitably, the formed microspheres are subject to an extended curing period in the acidification bath, for a period of at least 15 minutes (after gelation), and preferably for a period of at least 20 minutes. In a preferred embodiment of the invention, the formed microspheres are cured for a period of time from 20 to 180, 20 to 120, or 20 to 60 minutes. Ideally, the acidic curing solution is agitated during the curing process.

The microspheres of the invention are typically capable of surviving intact during passage through the mammalian stomach and capable of releasing the ferrous iron in the gastrointestinal tract distally of the stomach, for example in the small intestine. The term "surviving intact in the stomach" means that the microspheres are resistant to gastric and peptic break-down and release of iron in the gastric conditions of the mammalian stomach during gastrointestinal transit.

A preferred method of producing the microdroplets is a vibrating nozzle technique, in which the denatured de-calcified protein and iron salt are prepared separately and not mixed until just prior to or during extrusion through a nozzle and laminar break-up of the extruded laminar jet is induced by applying a sinusoidal frequency with defined amplitude to the spray nozzle. Examples of vibrating nozzle machines are the ENCAPSULATOR (Inotech, Switzerland) and a machine produced by Nisco Engineering AG, or equivalent scale-up version such as those produced by BRACE GmbH and the like.

Typically, the nozzle has an aperture of between 60 and 250 microns, preferably between 100 and 200 microns, suitably 140 and 160 microns, and ideally about 150 microns.

Suitably, the frequency of operation of the vibrating nozzle is from 900 to 3000 Hz. Generally, the electrostatic potential between nozzle and acidification bath is 0.15 to 0.3 V. Suitably, the amplitude is from 4.7kV to 7kV. Typically, the falling distance (from the nozzle to the acidification bath) is less than 50cm, preferably less than 40cm, suitably between 20 and 40cm, preferably between 25 and 35cm, and ideally about 30cm. The flow rate of suspension (passing through the nozzle) is typically from 3.0 to 10 ml/min; an ideal flow rate is dependent upon the nozzle size utilized within the process.

In one embodiment, the process involves a step of detecting the size of the initial microspheres generated, comparing the detected size of the microspheres with a predetermined desired size, and wherein the detected size differs significantly from the predetermined desired size, the microspheres are diverted away from the acidification bath until the detected size correlates with the predetermined desired size.

In this specification, the term "composition" should be understood to mean a composition of matter that comprises microspheres of the invention. Suitable compositions include comestible products such as food products and beverages, and food supplements in any form, for example unit dose products, powders, and the like. Typically food products include health drinks, yoghurts and yoghurt drinks, health bars, and the like.

The preparation of microspheres of the invention may be provided in a dried form, for example a spray-dried, drum dried, dehydrated, or freeze dried form, or they may be provided as a suspension is a suitable solvent, for example water.

Denatured de-calcified whey protein isolate (WPI) is preferable for the generation of robust encapsulation matrices and also contains an ideal protein gelation character in order to achieve controlled release or ferrous (Fe²⁺) molecules from gelled protein encapsulation microspheres, with a spherical diameter less than 250 microns. Whey protein concentrate (WPC) is also a possible encapsulation material however, higher concentrations are required to adjust for the low (but significant) levels of fat, cholesterol and carbohydrate, in the form of lactose.

One aspect of this technology involves the use of denatured de-calcified whey protein isolate /concentrate for the efficient encapsulation of iron supplements, ideally ferrous (Fe²⁺) supplements. This represents a novel processing step for commercial encapsulation of iron for targeted bioavailability. Commercial WPI has low (but significant) levels of calcium; hence it is preferably removed in order to generate an optimum encapsulation material for iron encapsulation. Hence, this processing step is preferred for i) efficient iron compound encapsulation and ii) enhanced bioavailability of iron within the blood serum of mammals.

In general, when iron and milk proteins are declared on the label together, iron absorption will be compromised as a result of calcium ions present in the dairy source. Hence, the efficient removal of calcium ions provides a viable solution for commercial iron encapsulation and targeted delivery to the blood serum. Functional denatured de-calcified whey protein microspheres have shown the ability to stabilise and deliver iron in the blood serum of mammals.

This initial process step involves WPI treatment by ion exchange to replace calcium with monovalent cations and dried to form a proteinaceous ingredient useful for the preparation of encapsulated ferrous iron supplements. Methods of de-calcification include (i) cation exchange on an ion exchanger charged substantially with a single species of monovalent cation (<90% of the exchangeable ions is a single species such as Hydrogen) (ii) acidification to pH 4.6-7 with subsequent dialysis and/or ultrafiltration and/or diafiltration or (iii) by addition of a chelating agent and/or binding a proportion of calcium ions (ionic or colloidal calcium) with a chelating or sequestering agent. In this context of ferrous encapsulation, it is understood that a small proportion of the cations bound to a cation exchange resin may be resistant to exchange with the desired cation

An alternative methodology for the calcium-depleted WPC for ferrous iron encapsulation applications is prepared comprising:
(a) providing a low fat milk solution, for example whey protein concentrate, in liquid form;
(b) removing of calcium ions therein by a method chosen from at least of (i) cation exchange on an ion exchange in a form bearing a monovalent cation species, or (ii) acidification to pH 4.6-7 optionally with subsequent dialysis; and
(c) concentrating the solution obtained by ultrafiltration, optionally with diafiltration, to form WPI having at least 90% dry weight of protein.

The calcium-depleted WPI is suitably dried and then re-dissolved in the optimum composition for ferrous encapsulation purposes. De-calcified whey protein isolate (WPI) used to form the ferrous microspheres, was preferably initially denatured at appropriate environmental conditions (pH, salt, solid concentration) to enable the production of a soluble dispersion of protein aggregates suitable for extrusion and encapsulation in the presence of sodium acetate and ferrous sulfate. This invention can be used to stabilize ferrous compounds in the matrix network whey protein microspheres. This process occurs instantaneously when whey protein comes into contact with ferrous sulfate at defined conditions of salt, pH and temperature.

The preparation of de-calcified whey protein (i.e. WPI) to form ferrous encapsulation material typically involves:
1. Dispersion of de-calcified WPI in water with concentrations in the range of 4- 30 % (w/w), more preferably between 7 - 30% (w/w), ideally between 9-16% (w/w). This may be achieved using high shear stirring in a blade mixer or Ultra-Turrax in the presence of a surfactant in the range of 0.01- 0.1% (w/w) ideally in the range 0.04-0.09% w/w) with a pH in the range of 5.0-9.0, ideally in the pH range 6.0 -7.0
2. Application of filtration to remove any denatured material with filtration pore size between 5 micron and 0.2 micron, more preferably between 0.45 microns and 1.2 micron. This may be achieved by sterile vacuum filtration.
3. Application of heat treatment to expose the specific hydrophobic sites in the protein material to enable controlled ferrous encapsulation. Protein denaturation is suitably performed between 60-140 degrees Celsius, preferably between 70-121 degrees Celsius at pH in the range of 5.0- 8.5, preferably in the range of 6.0-8.2. This process is entitled "protein activation" since it exposes protein regions to enable iron encapsulation and disulfide bond formation for protein polymerization in denatured structures.

Protein denaturation will unfold the globular whey protein molecule to reveal free thiol groups that are required for protein polymerization and gelation. For this embodiment of the invention, Ellman's reagent (2-nitro-5-mercaptobenzoic acid, DTNB) was utilised to determine the number of free thiol groups available after protein denaturation. The presented assay was performed in the absence of any reagents such as urea or SDS, because under these conditions, only the thiol groups of interest, those of the surface of the protein aggregate, were determined for encapsulation purpose. The number of thiol groups free for ferrous encapsulation was calculated using a molar extinction coefficient for DTNB of 13,600 M⁻¹ cm⁻¹.

The turbidity of the denatured de-calcified whey protein suspension was also an important test utilised to verify the optimum heat required to fully denature whey protein solutions. Turbidity measurements were performed at 25°C on a Cary Spectrophotometer (Varian, Netherlands) equipped with a temperature control unit. The turbidity was measured at 500 nm in quartz cuvettes with a path length of 2mm.

The denatured protein suspension is generally aseptically extruded through a nozzle into a tempered (45°C) sodium acetate bath (pH 4.0, 0.2M) with 25 mg ferrous sulfate. The acetate-ferrous gelling bath is typically continuously agitated to avoid coalescence or flocculation of microspheres during protein polymerization i.e. sphere formation. Pliable microspheres were generally polymerized at room temperature, recovered and suitably dispersed in tri-sodium citrate (0.4M) and subsequently washed with sterile water. This method represents protein polymerization as a result of ionic gelation of de-calcified denatured whey protein i.e. denatured protein gelation in the presence of ferrous sulfate in acetate solution.

Alternatively, the de-calcified denatured protein suspension can be extruded through a concentric nozzle with 25mg (±0.334mg) of ferrous sulfate (no flocculation) into an sodium acetate gelling bath (pH 4.0, 0.2M) with continuously agitation to avoid coalescence or flocculation of microspheres during polymerization i.e. sphere formation. Pliable microspheres were polymerized at room temperature, recovered and dispersed in tri-sodium citrate (0.4M) and subsequently washed with sterile water. This method represents protein polymerization as a result of isoelectric focusing i.e. protein gelation at pH conditions close the isoelectric point of denatured de-calcified whey protein.

The above parameters provide the optimum denatured globular protein rheological property for use of de-calcified denatured WPI as an encapsulation material of this specific ferrous invention. Rheological properties include gel strengths, viscosity and stress-strain characteristics when subject to either static or dynamic deformation.

Formation of microspheres typically involves use of an encapsulator. Suitably, the iron encapsulator consists of a single orifice (Fig. 1e) for transition of the extruded de-calcified denatured whey protein. A precision-drilled sapphire stone is fitted at the orifice, on the tip of a stainless steel cone to avoid imperfections in nozzle geometry cause by utilizing cheaper alternatives. Microspheres in the size range of 60 microns

(µm) to 250 microns µm can be generated using sterile and non-sterile conditions. Production of mono-dispersed microspheres with a very narrow size distribution involves the generation of a steady flow of the de-calcified denatured whey protein through the nozzle at controllable rates, which will provide optimum breakup of the extruded liquid jet into droplets (Fig. 1k). The system flow rate is governed by two systems, which depend on the required micropshere quantities. The work performed in this invention involved a pulsation-free high precision syringe pump (Fig. 1a) to extrude 1 Litre Whey Protein batches. In order to facilitate extrusion of a continuous stable laminar liquid jet through the nozzle, a system pre-requisite outlines the use of a high flow rate to surpass the viscosity and /or the surface tension of whey protein. If ferrous sulfate is mixed with whey protein prior to this step, temporary stability would be achieved; however, long term ferrous stability would not be achieved due to the pH of the original reaction (pH 7). It has been demonstrated that ferrous (iron) is stabilized at pH <4.5. In this way, this invention encapsulates ferrous (iron) within extruded iron droplets, at pH 4.0 in acetate buffer, 0.2M, 45°C. Hence, the flow rate is not excessively high, which avoids i) inefficient jet-breakup of whey protein; ii) enhanced impact forces on the whey protein droplets when entering the iron gelling bath; iii) unpredictable deformation and iv) increased probability of coalescence.

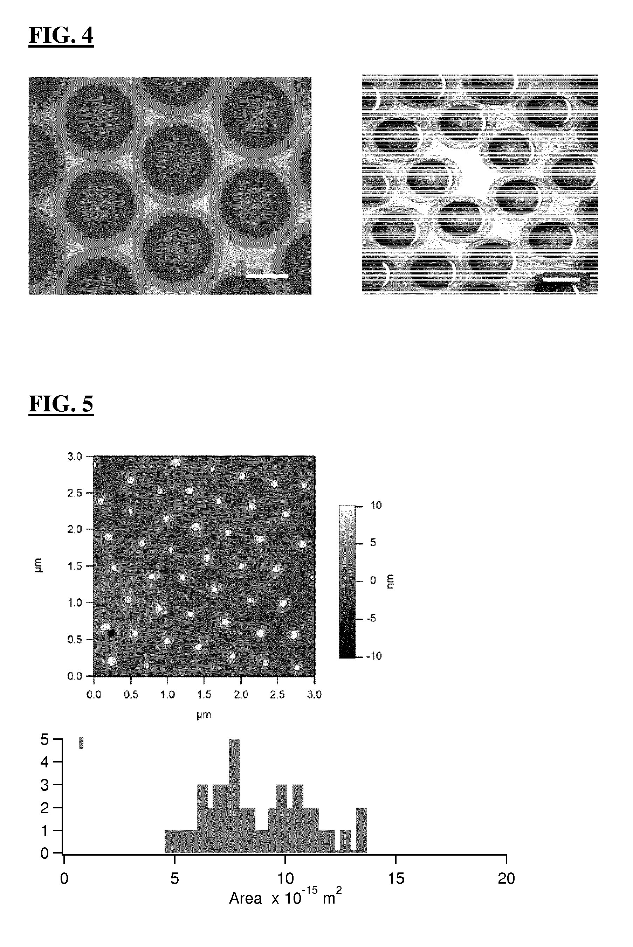

Microsphere size can be varied within a certain range by regulating the frequency of vibration and /or the flow rate of the polymer with higher frequencies and lower jet velocities enabling the generation of smaller microspheres of entrapped Ferrous. Nonetheless, the main factor governing microsphere size the nozzle diameter (Fig. 4e), whereby the final microsphere diameter is approximately 1.25X the size of the chosen nozzle.

Optimal breakup of the jet results in the formation of a single bead chain, which can be monitored using a stroboscopic lamp (Fig. 1g) placed directly behind the chain providing visualization of individual drops during breakup. De-calcified denatured whey protein droplet coalescence in the laminar jet stream will result in loss of monodispersity and an increase in the microsphere size deviation. To avoid coalescence in the air, a strong negative charge is induced on the de-calcified denature Whey Protein droplet surface using an electrostatic voltage system, which generates droplet repulsion and dispersal of the chain into a cone like shape (Fig. 1k) due to Coulomb forces. Tangential deflection of the jet stream during breakup prevents 'doublet' microsphere formation and also enlarges the drop-zone in the hardening solution (Fig. 1l) resulting in mono-dispersed, homogenous and spherical microspheres of encapsulated Ferrous sulfate.

Droplet entry into the polymerization solution with ferrous is of paramount importance for microsphere morphology. Upon landing in the agitated bath, droplets are gelled by ionotropic gelation with acetate buffer in the presence of iron. Surface tension issues were overcome by the addition of a surfactant (Tween-20), a common food ingredient. This practical approach significantly reduced surface tension and reduced the incidence of non-spherical microspheres and coalescence by avoiding the delayed departure of Ferrous droplets from the gelling solution surface.

Monodisperse microspheres were prepared for encapsulation of Ferrous Sulfate using de-calcified denatured whey protein dispersions. In the present invention, the Whey Protein reservoir was delivered to the nozzle via a feed line utilizing nozzles with 40-100 µm diameters. The nozzle was connected via a PTFE membrane to a vibrating device, which was insulated from the surrounding structures by rubber mounts to avoid the generation of resonance frequencies in the system. The flow of the Protein dispersion to the nozzle was accomplished using a peristalic pump with maximum extrusion volume of 1 Litre. The production of < 1 litre of microspheres was sufficient to meet the requirements of this preliminary study; hence the encapsulator resembled a batch-reactor on this occasion only. Sterile protein suspensions were equilibrated to room temperature. The denatured Whey Protein suspension was aseptically extruded through a nozzle into a tempered (45°C) sodium acetate bath, pH 4.0, 0.2M, with 25 mg ferrous sulfate. Protein polymerization at this specific temperature will induce the production of physical interactions such as hydrophobic or Van der Waals interactions. The gelling bath was continuously agitated to avoid coalescence or flocculation of microspheres during polymerization i.e. sphere formation. Pliable microspheres were polymerized at room temperature, recovered and dispersed in tri-sodium citrate and subsequently washed with sterile water. The acetate buffer conditions (0.2M; 45°C; pH 4.0) are favorable for iron encapsulation since the i) pH is optimum for ferrous stability; ii) the temperature is favorable for iron solubility; and iii) buffer concentration is optimised to enable the entrapment and stability of divalent cations, such as ferrous sulfate. A subsequent curing step in a tri-sodium citrate buffer (0.3M; 20°C; pH 5.0) further enhances iron bioavailability and bioaccessability in the blood serum.

### Experimental

### Generation of microspheres

### (a) De-calcification of whey protein

This initial process step in the presented invention involves WPI treatment by ion exchange resins to replace calcium with monovalent cations for the preparation of whey protein for encapsulation purposes. The current invention utilized WPI treated with a single (<90% of the exchangeable ions is a single species such as Hydrogen) or mixed resins (where large proportion i.e. approx. 60% of the resin is charged with hydrogen ions and the significantly lower proportion i.e. 40% is treated with sodium ions to produce a de-calcified WPI. In the present invention, approx. 90% resin is charged with strongly acidic groups, preferably sulfonate groups. The functional groups are sulphonic acid groups that can be obtained in the Na⁺ form or alternatively converted to the K⁺ form to produce a de-calcified WPI with optimum encapsulation properties. This invention utilised a cation exchange on an ion exchanger charged substantially with a single species of monovalent cation (<90% of the exchangeable ions is a single species such as Hydrogen). The WPI applied to the cation exchanger preferably has the pH in the range of 5.0 - 7.0. When the void volume is eliminated and the column is running the WPI solution, the pH requires regular adjustment to pH 6.5-7.0 using 0.2M HCl. The following step involves acidification using citrate (0.2M) to pH 4.6-7.0 and filtration (through 0.45 micron) to remove any denatured material generated during ion exchange. The next step involves ultrafiltration using a membrane with 10,000 Daltons or less Molecular weight cut-off (MWCO).

### (b) Encapsulation of ferrous iron - Example 1

Calcium-depleted WPI is dried and then re-dissolved in the optimum composition for ferrous encapsulation purposes. The ferrous iron encapsulation system was prepared using the calcium-depleted WPI preferably contains (per 100gram) from 125mg - elemental iron and 95 gram protein, in absence of calcium, Microspheres composed of a ferrous sulfate gel core and a hydrogel membrane were prepared using the coextrusion jet- break- up technique. A stock solution of whey protein was prepared in phosphate buffer (pH 7; 2M) in a blade mixer or Ultra-Turrax in the presence of a surfactant in the range of 0.01- 0.1% (w/w) at pH range 6.0-7.0. The solution is filtered through a 0.45 - 0.2 microns under a pressure of 4-65 bar prior to ensure the removal of denatured protein material generated during powder processing. Whey protein isolate (WPI) was subsequently heat-denatured at appropriate environmental conditions (pH 7.0, >78°C; 4-9 % w/w protein content). Heat treatment is performed ideally between 70-140°C at pH in the range of 5.0-8.5. Heat denaturation is performed under agitation (150-200 rpm) to enable the production of a soluble suspension of protein aggregates suitable for ferrous encapsulation. Heat denaturation is performed for a min. 30 minutes / max. 90 minutes to allow full denaturation and exposure of specific hydrophobic sites in the whey protein molecule. It is noteworthy that the protein requires full denaturation; this process fully denatures whey protein to permit the exposure and "unfolding" of hydrophobic sites on the globular protein structure for optimum ferrous encapsulation. This step is entitled the "Protein Activation" due to the fact that it "activates" whey protein for an inventive ferrous encapsulation process via denaturation processes.

After Protein Activation (i.e. heat denaturation), the solution of aggregates ware rapidly cooled to 20°C using ice. The solutions are then diluted to approx. 5-9% (w/w) protein concentrations (using MilliQ water) before modification and stored at 4°C for 6-8 hours with no agitation.

After heat denaturation, dilution and specified storage, the protein suspension was adjusted to optimum pH value to generate strong negative charge on the protein particles (i.e. >pH 4.8, ideally greater than pH5.1, the isoelectric point of β-Lactoglobulin (β-Lg). Following protein equilibration, the suspension of negatively charged protein aggregates are aseptically extruded through a single nozzle into a tempered sodium acetate bath (45°C; pH 4.0, 0.2M), containing 25 mg ferrous sulfate/ 200mL. Spherical microspheres were obtained by the application of a vibrational frequency with defined amplitude to the co-extruded jet and collected in a acetate gelling placed 18 cm below the nozzle and agitated by a magnetic stirrer (length 4cm) in dish with diameter 35 cm. Polymer flow rate and vibration frequency were empirically determined for the specific viscosity & concentration of denatured whey protein in the "activated", negatively charged form. The appearance of a protein gelation upon contact with gelling bath, occurred at a minimal de-calcified denatured protein concentration of 1.75% (9% diluted to 1.75%) induced by protein aggregation and non-covalent chemical interactions. For the purpose of optimum ferrous encapsulation in homogenous, spherical microspheres, (for delivery to the duodenum and upper jejunum) the protein concentrate must be greater than 3% (w/w) de-calcified whey protein at pH greater than 5.1 to promote physical interactions such as hydrophobic and van der Waals interactions within the ferrous protein network. The acetate-ferrous gelling bath is continuously agitated to avoid coalescence or flocculation of microspheres during protein polymerization i.e. bead formation and ferrous encapsulation. Pliable microspheres were agitated (50 rpm) at room temperature in the acetate bath for minimum 30 minutes. These microspheres are then recovered and dispersed in tri-sodium citrate (0.4M) and subsequently washed with sterile water. This step sequesters calcium ions from the local environment in order to avoid ferrous contact with calcium ions upon microsphere digestion and ferrous release. After extrusion and chemical cross-linking of the protein polymer membrane in acetate and citrate, the microspheres were used immediately autoclaving at 121°C for 15 minutes for sterilisation. This method represents whey protein polymerization as a result of ionic gelation i.e. protein gelation in the presence of ferrous sulfate in acetate buffer solution.

### (b) Encapsulation of ferrous iron - Example 2

Similar to the aforementioned method i.e. protein de-calcification, heat denaturation and preparation, the suspension of negatively charged protein aggregates were aseptically extruded through a concentric nozzle into a tempered (45°C) sodium acetate bath (pH 4.0, 0.2M). For this reason, the encapsulator was fitted with concentric nozzle with an internal diameter of 80µm and an external diameter of 100 µm. Two syringe pumps (200 series, KD Scientific, Boston, USA) were connected to the encapsulator to supply ferrous sulfate through the central nozzle and de-calcified denatured protein solution through the external nozzle. The inner nozzle contained de-calcified denatured whey protein suspension with a slightly higher protein concentration for gelation. Spherical microspheres were obtained by the application of a vibrational frequency with defined amplitude to the co- extruded jet and collected in a acetate gelling bath (which may alternatively contain the ferrous sulfate placed 18 cm below the nozzle and agitated by a magnetic stirrer (length 4cm) in a dish with diameter 40 cm. Polymer flow rate, ferrous flow rate and vibration frequency were empirically determined for the specific viscosity & concentration of whey protein (in the denatured, negatively charged form) and ferrous sulfate at bioavailable pH conditions (<4.5). The appearance of a protein-iron gelation upon contact with acetate gelling bath, occurred at a minimal de-calcified denatured protein concentration of 2.5% (9% diluted to 2.5%) induced by protein aggregation and non-covalent chemical interactions. For the purpose of optimum ferrous encapsulation using a concentric nozzle system, the protein concentration must be greater than 5.5% (w/w) de-calcified whey protein at pH greater than 5.1 to promote the generation of homogenous, spherical microspheres for duodenum and upper jejunum delivery, with suitable physical interactions i.e. hydrophobic and van der Waals interactions within the ferrous protein network (interactions are temperature dependent). Pliable microspheres were polymerized at room temperature, recovered and dispersed in tri-sodium citrate (0.4M) and subsequently washed with sterile water. This step sequesters calcium ions from the local environment in order to avoid ferrous contact with calcium ions upon microsphere digestion and ferrous release. After extrusion and chemical cross-linking of the polymer membrane in acetate and citrate, the capsules were used immediately autoclaving at 121°C for 15 minutes. This method represents protein polymerization as a result of isoelectric focusing i.e. protein gelation at pH conditions close the isoelectric point of de-calcified denatured whey protein.

### Characterisation of microspheres

Image analysis was performed using a Leica TCS SP5 confocal scanning laser microscope (CSLM) (Leica Microsystems, Wetzler, Germany) for the purpose of micro-capsule morphology and porosity assessment within the micro-structure. Micro-capsules with encapsulated ferrous sulfate were stained using the Nile Red (BD Biosciences, San Jose, California), by integrating dye concentrations optimized for ferrous detection. Thickness of the optical section was in the order of several micrometers. Changing the plane of focus vertically up and down by 10 µm had no effect on the apparent membrane thickness. Image analysis was performed using ×63 magnification objective with numerical aperture of 1.4. Confocal illumination was provided by an argon laser (488 nm laser excitation) and red-green-blue images (24 bit), 512 by 512 pixels, were acquired using a zoom factor of 2.0, giving a final pixel resolution of 0.2 µm /pixel.

Atomic force microscopy (AFM) images were also obtained using Asylum Research MFP-3D-AFM (Asylum Research UK Ltd., Oxford, UK) to investigate the morphology i.e. depth, width and frequency of pores on the surface of the microspheres.. The microspheresare poured onto microscope glass slides, incubated for slide adsorption for approx. 20 minute, washed with ultraclean water and dried in a nitrogen flow leading to a linear surface coverage of microspheres on the slide. Suitable microsphere samples could then be chosen in transmitted light DIC contrast prior to AFM scanning. The AFM head is then placed onto a Zeiss Axiovert 200 inverted optical microscope. AFM analysis was relatively slow since microspheres ware delicate and easily damaged mechanically if the force exerted by the AFM tip was too high. Microscope images were obtained in intermittent contact mode with standard silicon non-contact cantilevers (NCH) provided by Asylum Research, UK. Resonance frequency was approx. 200 kHz and spring constant of 30N/m. Digital resolution was 512 x 512 pixels. The tip radius of cantilever was less than 10 nm and the opening angle between 30° and 40°. The applied force between the cantilever tip and ferrous microspheres was minimised in order to alleviate the influence of the AFM tip on the microspheres surface topography during pore scanning. Measurements were started by scanning a random area of 1 m, with subsequent zoom to 20nm. These images were used to evaluate the morphology of microspheres pores. Scan size was decreased gradually until microsphere pores could be viewed clearly. Iron-whey protein microspheres were analysed and scanned using typical working distance of 2-6 nm for visualization of pores dimensions and general morphology.

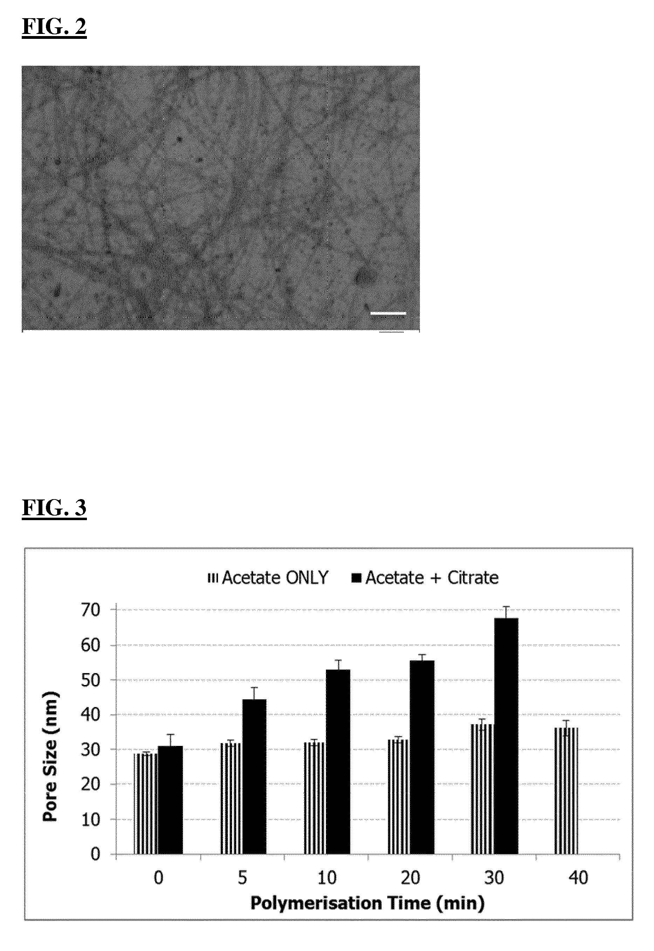

The gel structure of ferrous- protein microspheres exhibited a repeating network of ferrous cavities and protein strands, with diameter ranging 22-58 nm, is shown in Fig. 2. For microspheres gelled in acetate, the measured thickness of the protein strand in the microsphere membrane was approx. 3.74 ± 0.2 nm. However, when an additional citrate bath was incorporated, the microsphere membrane thickness was ideal for targeted microsphere digestion in the duodenum and upper jejunum (see Fig. 3). Work has shown that calibration of microsphere membrane thickness (with acetate and citrate coatings) has a direct relationship with target release location in the intestine of the mammal.

This AFM technique is optimally calibrated to determine the release location of produced microspheres with added acetate and citrate coatings. Fig. 4 illustrates microspheres with ferrous sulfate encapsulated within de-calcified denatured whey protein (with acetate and citrate coatings) for targeted release in the duodenum and upper jejunum of the mammalian intestine.

Atomic Force Microscopy has the ability to screen membrane porosity. This present invention reveals (the first) AFM screening of porosity on the surface of microspheres. This inventive technique provides a tool for qualitative and quantitative analysis demonstrates of pore distribution (i.e. homogenous or sporadic) across the microspheres surface. Fig. 5 shows the calibration grid utilised in this invention to estimate the pore size and frequency, which is directly related to microspheres release and digestion location.

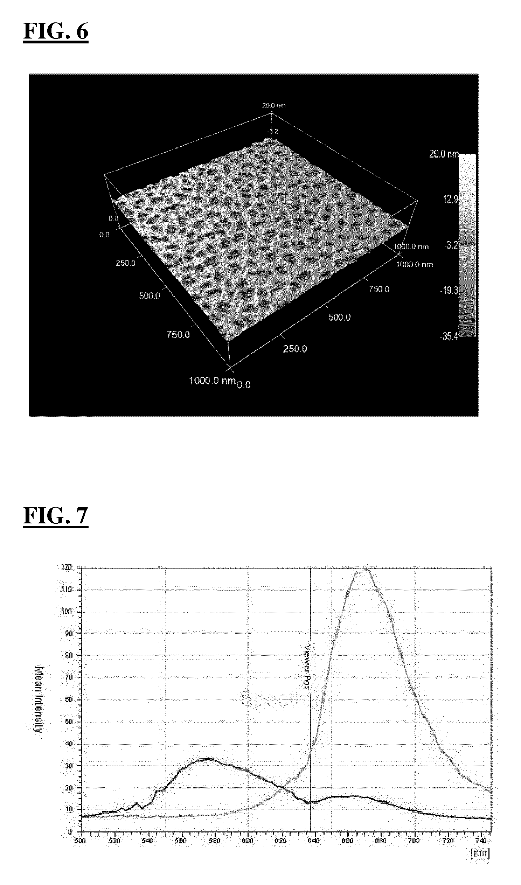

Microscopy analysis demonstrated the presence of deep pore cavities on microspheres surfaces after both acetate and citrate treatments. Fig. 7 illustrates the lack of pores after acetate treatment (purple line), while large crevices are generated after acetate and citrates polmerisation bathes (green line). This reveals the muco-adhesive properties generated as a result of the two polymerization baths for enhanced bioavailability of ferrous sulfate in the mammalian blood serum.

### In-vivo-data

### B:

A prospective, randomized, cross-over, single-blind, cross-over study with at least 1 week wash-out was carried out to evaluate the pharmacokinetics (% relative bioavailability, total serum iron AUC₀₋₄ₕ (µM.h)) of a single oral dose of the invention compared to the same dose of a market leading oral iron formulation. The single oral dose was made up of 15g of microspheres of the invention (dry weight) made according to Example 2 above, suspended in 200mls of apple juice, in which the 15g of microspheres included 25mg (± 0.334mg) of iron. We excluded subjects taking iron supplements and those with haemoglobin >11.5 g/dL, serum ferritin > 150 µg/L, haemochromatosis or other iron storage disorders, gastro-intestinal disorders, inflammatory bowel disease, any medications and any intolerance to any of the components of either formulation.

Each dose was taken orally in the fasting state (>8 hours) as a drink, after fasting since midnight the night before the study. Blood samples were obtained over a 4 hour period in the fasting state (in all cases baseline, 2, 4 hours post dose). During this time they were not permitted to drink tea or coffee, but are allowed to drink water ad libitum an hour after the initial sample is taken.

Samples were taken by a trained, qualified nurse and all evaluations were performed under medical supervision. Samples were analysed for serum iron, total iron binding capacity (TIBC) and serum ferritin. Baseline venous blood samples were also drawn to obtain a full blood count and measure and haemoglobin levels prior to inclusion and administration of the test articles. All analyses were carried out in a blinded fashion by an externally validated esoteric laboratory operating under INAB accreditation. The primary endpoint of the study was the AUC0-4h of total serum iron (µM.h) over 4 hours post-dose. The results (n=3) are presented in Figure 8. Statistical analyses were carried out using paired sample t-tests. The data show that the serum iron level achieved with the present invention is significantly greater than with the market leading formulation (Figure 8a) and that the response is consistent across all subjects (Figure 8b). The % relative bioavailability of microencapsulated oral iron (ST1302) is estimated at 468% compared to the market leading oral iron formulation.

Participants also completed a questionnaire on the taste and acceptability (including adverse effects) of the supplements (See Fig. 9).

The invention is not limited to the embodiments hereinbefore described which may be varied in construction and detail without departing from the spirit of the invention.

### References

[1] Muñoz, Manuel, Isabel Villar, and José Antonio Garcia-Erce. "An update on iron physiology." World journal of gastroenterology: WJG 15.37 (2009): 4617.
[2] Lieu, Pauline T., et al. "The roles of iron in health and disease." Molecular aspects of medicine 22.1 (2001): 1-87.
[3] Dubois, S. (2013) Physiological Function of Iron in Humans. Available at: http://woman.thenest.com/physiological-function-iron-humans-6476.html (Accessed 31 January 2014)
[4] Bothwell TH. Overview and mechanisms of iron regulation. Nutr Rev 1995;53(9):237-45
[5] Green R, Charlton R, Seftel H, et al. Body iron excretion in man: a collaborative study. Am J Med 1968;45:336-53
[6] Reviewed in Jankowska E, von Haehling S, Anker S et al. European Heart Journal (2013) 34, 816-826
[7] Zhang AS, Enns CA. Molecular mechanisms of normal iron homeostasis.
[8] Anderson GJ, Frazer DM, McLaren GD. Iron absorption and metabolism. Curr Opin Gastroenterol 2009;25:129-135.
[9] Anderson GJ, Vulpe CD. Mammalian iron transport. Cell Mol Life Sci 2009;66:3241-3261
[10] Munoz M, Garci'a-Erce JA, Remacha AF. Disorders of iron metabolism. Part 1:molecular basis of iron homoeostasis. J Clin Pathol 2011;64:281-286. Hematology Am Soc Hematol Educ Program 2009;1:207-214.
[11] Hematology Am Soc Hematol Educ Program 2009;1:207-214. Hentze MW, Muckenthaler MU, Galy B, Camaschella C. Two to tango: regulation of Mammalian iron metabolism. Cell 2010;142:24-38
[12] Wang J, Pantopoulos K. Regulation of cellular iron metabolism. Biochem J 2011; 434:365-381
[13] Babitt JL, Lin HY. Molecular mechanisms of hepcidin regulation: implications for the anemia of CKD. Am J Kidney Dis 2010;55:726-741.
[14]Viatte L, Vaulont S. Hepcidin, the iron watcher. Biochimie 2009;91:1223-1228.
[15]Franchini M, Montagnana M, Lippi G. Hepcidin and iron metabolism: from laboratory to clinical implications. Clin Chim Acta 2010;411:1565-1569.
[16] Nemeth E, Ganz T. The role of hepcidin in iron metabolism. Acta Haematol 2009; 122:78-86.
[17] Handelman GJ, Levin NW. Iron and anemia in human biology: a review of mechanisms. Heart Fail Rev 2008;13:393-404
[18] European Commission (2012) Commission Regulation (EU) No 432/2012. Luxembourg: official Journal of the European Union
[19] Batool A Haider, Ibironke Olofin, Molin Wang, Donna Spiegelman, Majid Ezzati, Wafaie W Fawzi. Anaemia, prenatal iron use, and risk of adverse pregnancy outcomes: systematic review and meta-analysis. BMJ 2013;346:f3443 doi: 10.1136/bmj.f3443
[20] Persson LÅ, Arifeen S, Ekström EC, Rasmussen KM, Frongillo EA, Yunus M. Effects of prenatal micronutrient and early food supplementation on maternal hemoglobin, birth weight, and infant mortality among children in Bangladesh: the MINIMat randomized trial. JAMA. 2012;307(19):2050-9.
[21] Haider BA, Yakoob MY, Bhutta ZA. Effect of multiple micronutrient supplementation during pregnancy on maternal and birth outcomes. BMC Public Health. 2011;11(suppl 3):S19.
[22] WHO Micronutrient deficiencies series: Iron deficiency anaemia. www.who.int/nutrition/topics/ida/en/ accessed 7.7.13.
[23] Institute of Medicine, Food and Nutrition Board. Dietary Reference Intakes for Vitamin A, Vitamin K, Arsenic, Boron, Chromium, Copper, Iodine, Iron, Manganese, Molybdenum, Nickel, Silicon, Vanadium and Zinc. Washington, DC; National Academy Press, 2001.
[24] National Institute of Health (2007). Iron Dietary Supplement Fact sheet. Available at: http://ods.od.nih.gov/factsheets/Iron-HealthProfessional/#en1 (Accessed 31 January 2014)
[25] Skikne B, Baynes RD. Iron absorption. In: Brock JH, Halliday JW, Pippard MJ, Powell LW, eds. Iron metabolism in health and disease. London, UK: W.B. Saunders, 1994:151-87
[26] Hallberg L. Bioavailability of dietary iron in man. Annu Rev Nutr 1981;1:123-47.
[27] Stoltzfus R, Dreyfuss M. Guidelines for the use of iron supplements to prevent and treat iron deficiency anaemia.
[28] Besarab A, Coyne DW. Iron supplementation to treat anemia in patients with chronic kidney disease. Nat Rev Nephrol 2010;6:699 -710.
[29] Charytan C, Qunibi W, Bailie GR; Venofer Clinical Studies Group. Comparison of intravenous iron sucrose to oral iron in the treatment of anemic patients with chronic kidney disease not on dialysis. Nephron Clin Pract 2005;100:c55- c62
[30] Peña-Rosas JP, De-Regil LM, Dowswell T, Viteri FE. Intermittent oral iron supplementation during pregnancy. Cochrane Database Syst Rev. 2012;7:CD009997. doi: 10.1002/14651858.CD009997.
[31] Halksworth G, Moseley L, Carter K, Worwood M. Iron absorption from Spatone (a natural mineral water) for prevention of iron deficiency in pregnancy. Clin Lab Haematol. 2003;(4):227-31.
[32] Berit Borch-Iohnsen, Gunnar Bakkene, Marianne Ekman, Peter Reizenstein, High bioavailability to humans of supplemental iron in a whey concentrate product. Nutrition Research, Volume 14, Issue 11, November 1994, Pages 1643-1648
[33] Zhang D, Mahoney AW. Bioavailability of iron-milk-protein complexes and fortified cheddar cheese. J Dairy Sci. 1989 Nov;72(11):2845-55.
[34] Hurrell RF, Lynch SR, Trinidad TP, Dassenko SA, Cook JD. Iron absorption in humans as influenced by bovine milk proteins. Am J Clin Nutr. 1989 Mar;49(3):546-52.
[35] Remondetto GE, Beyssac E, Subirade M. Iron Availability from Whey Protein Hydrogels: An in Vitro Study J. Agric. Food Chem. 2004, 52, 8137-8143 8137.
[36] Tomoki Nakano, Tomomi Goto, Tarushige Nakaji and Takayoshi Aoki1 Bioavailability of Iron-fortified Whey Protein Concentrate in Iron-deficient Rats. Asian-Aust. J. Anim. Sci. Vol. 20, No. 7 : 1120 - 1126
[37] Ou K, Liu Y, Zhang L, Yang X, Huang Z, Nout MJ, Liang J. J Agric Food Chem. Effect of neutrase, alcalase, and papain hydrolysis of whey protein concentrates on iron uptake by Caco-2 cells. 2010 Apr 28;58(8):4894-900. doi: 10.1021/jf100055y.
[38] Prusse, U., Bilancetti, L., Bucko, M., Bugarski, B., Bukowski, J., Gemeiner, P., Lewinska, D., Manojlovic, V., Massart, B., Nastruzzi, C., Nedovic, V., Poncelet, D., Siebenhaar, S., Tobler, L., Tosi, A., Vikartovska, A., Vorlop, K.D. Chemical Papers. Comparison of different technologies for alginate beads production. 2008: 62, 364-374.
[39] Haas, P.A., Industrial & Engineering Chemistry Research. Formation of uniform liquid-drops by application of vibration to laminar jets. 1992: 31, 959-967.
[40] Brandenberger, H., Nussli, D., Piech, V., Widmer, F., Journal of Electrostatics. Monodisperse particle production: A method to prevent drop coalescence using electrostatic forces. 1999: 45, 227-238.

## Claims

1. A preparation of microspheres comprising a multiplicity of discrete microspheres, in which the microspheres comprise iron entrapped within a protein matrix, and in which the protein in the protein matrix is at least partially denatured.

2. A preparation of microspheres as claimed in Claim 1, wherein the protein in the protein matrix has been subjected to divalent metal ion removal process.

3. A preparation of microspheres as claimed in Claim 1 or 2, wherein the protein in the protein matrix comprises whey protein, another milk protein, or pea protein.

4. A preparation of microspheres as claimed in Claim 1, 2 or 3, wherein the iron is ferrous iron, optionally selected from iron sulfate, iron chloride, iron ascorbate, and an iron carbohydrate complex.

5. A preparation of microspheres as claimed in any of Claims 1 to 3, in which the iron is a ferric (III) iron, optionally selected from iron (III) maltose or iron (III) maltol.

6. A preparation of microspheres as claimed in any preceding Claim, wherein the microsphere is a microsphere with a diameter of between 60 and 250 microns.

7. A preparation of microspheres as claimed in any preceding Claim, wherein the microsphere comprises a coating of a citrate, phosphate, or ascorbate salt.

8. A preparation of microspheres as claimed in any preceding Claim, wherein the protein of the protein matrix comprises denatured, de-calcified, whey protein.

9. A method of treating or preventing iron deficiency in a mammal, comprising the steps of administering a preparation of microspheres according to any of Claims 1 to 8 to the mammal.

10. A preparation of microspheres of any of Claims 1 to 8, for use as a dietary supplement in preventing the development of iron deficiency in a healthy mammal.

11. A composition comprising a preparation of microspheres in accordance with any of claims 1 to 8, in which the composition is selected from a food or beverage product, and animal feed, or a health supplement for humans or animals.

12. A method for making a preparation of microspheres according to any of claims 1 to 8, comprising the steps of:
a. preparing an iron-containing solution;
b. separately preparing a suspension of at least partially denatured protein;
c. delivering the solution and suspension to a nozzle;
d. simultaneously extruding the solution and suspension through the nozzle in a laminar jet in a manner that produces microdroplets; and
e. curing the microdroplets in an acidification bath such as an acetate bath.

13. A method for making a preparation of microspheres according to any of claims 1 to 8, comprising the steps of:
a. extruding a suspension of at least partially denatured protein through a vibrating nozzle to form a laminar jet in which break-up of the extruded laminar jet into microdroplets is induced by applying a sinusoidal frequency with defined amplitude to the nozzle; and
b. curing the microdroplets in an iron containing acidification bath to generate the microspheres comprising the iron entrapped within a crosslinked protein matrix.

14. A method according to Claim 14 in which the acidification bath comprises ferrous iron and/or in which the acidification bath comprises an acetate buffer having a pH of 3.5 to 4.5, a concentration of 0.15M to 0.25M, and optionally a temperature of 40-50°C.

15. A method as claimed in any of Claims 13 or 14, in which:
the microparticles are immersed in a buffer comprising a citrate or phosphate salt for a period of time sufficient to allow coating of the microparticless with a citrate or phosphate salt; and/or
in which the protein suspension or solution comprises 5-15 % de-calcified denatured whey protein (w/v).
